# EUROPEAN PATENT SPECIFICATION

(11) **EP 1 424 046 B1**
(45) Date of publication and mention of the grant of the patent: **28.02.2007**
(21) Application number: 02730297.5
(22) Date of filing: 25.04.2002
(51) Int. Cl.: A61F 2/12

(54) **BREAST PROSTHESIS**
BRUSTPROTHESE
PROTHESE MAMMAIRE

(30) Priority: 07.05.2001 ES 200101038
(43) Date of publication of application: 02.06.2004
(73) Proprietor: GONZALEZ DE VICENTE, Ricardo, 46183 La Eliana Valencia (ES)
(72) Inventor: GONZALEZ DE VICENTE, Ricardo, 46183 La Eliana Valencia (ES)
(74) Representative: Urizar Anasagasti, Jesus Maria
(86) International application number: PCT/ES2002/000202
(87) International publication number: WO 2002/089708

(56) References cited:
- EP-A1- 0 422 302
- US-A- 4 507 810
- US-A- 4 955 907
- US-A- 5 447 535
- US-A- 5 674 285

## Description

### OBJECT OF THE INVENTION

The object of the present patent of invention is to present a new breast prosthesis using a widely used and accepted material for these implants, such as silicone, but with a body with a micro-cell structure, either wrapped in a silicone covering or not, providing a lighter body with variable density with no liquid or gelatin components that can extravasate the limits of the coverings, leaking out, furthermore eliminating the risks of punctures, encapsulations, etc

### BACKGROUND OF THE INVENTION

To date, implanted breast prostheses essentially consist of two components: an outer one or a covering and an inner one or the filling. The outer one is internationally common for all trademarks and manufacturers of breast implants and is based on a vulcanized silicone covering of two, three or, in the best of cases, five layers, for the purpose of trying to decrease to a minimum the "migration" or transudation phenomenon of the implant, which consists of the implant filling product or substance (silicone, saline, hydrogel, etc.) passing through microscopic pores of the wall of the covering, with more or less success in each case.

Thus, for example:
US patent number US 4,507,810 A describes an implantable prosthesis formed by a series of cells of irregular sizes, interconnected between them and filled with a liquid material. The prosthesis described in said patent differs fundamentally from the present prosthesis because in this model there is no interconnection and there is no filling of liquid material, therefore, if there is a possible rupture or puncture, no liquid material would be spilled into the organism.
US patent number 4,955,907 A describes a texturized coating of material biocompatible with the organism, formed by a plurality of individual polygonal geometric shapes containing liquid, gas or gel. Manufacturing this product has a serious drawback from the point of view of content and a high risk of leakages and ruptures.
European patent EP 422,302 A1 and US patent 5,447,535 A refer to a prosthesis comprising a peripheric silicone membrane full of small individual closed cells filled with a biocompatible liquid. The cited prostheses differ in fundamental points with the present specification because they always refer to a container and a content with the subsequent aforementioned drawbacks.
US patent 5,674,285 A refers to an implant with a silicone outer surface layer having an open cell structure; it is made by applying solid interconnected particles with a variable diameter to the outer surface layer before the latter is cured. It is another type of prosthesis in which there is also a container and a content, with all the drawbacks that this entails.

Finally, it has to be indicated that currently, as has been previously described, the main drawback with traditional implants is that this migration results in a decrease of the initial volume of the patient's implant and, therefore, of the volume obtained after her operation in some cases or the total loss of volume in the case of implants with a filling valve mechanism. In the case of silicone gel implants, the silicone can migrate through different routes: fat, lymph nodes, etc., with unwanted results for the organism.

Some breast prostheses, when they are crushed by body weight such as when sleeping, with time end up losing volume, and therefore losing the reason for which they were implanted.

All current implants have a liquid or gel content that can extravasate upon bursting, they are also susceptible to punctures or ruptures when handled for the implanting or subsequent use thereof due to fatigue of the material with time, due to friction between folds and creases, etc., with the subsequent economic losses for the manufacturer, doctor, and the drawbacks for the patient in terms of economic drawbacks as well as discomforts upon second operations, touch-ups, etc, this effect being acute when a puncture is generated, as the amount of the migrated substance is greater.

Another of the problems caused by the "transuding" or migration of the inner component is encapsulation, in other words, hardness created by the irritation of the scar or wound remaining after the intervention in contact with these transmigrated particles from inside the prosthesis, causing a callosity which hardens the breast.

On the other hand, the weight of the prosthesis significantly increases the natural weight of the breast, therefore with time, the latter tends to sag and require new aesthetic operations, including replacement of the prosthesis, with the aforementioned subsequent drawbacks.

### DESCRIPTION OF THE INVENTION

The new breast prosthesis object of the present invention is proposed to prevent or lessen all the aforementioned drawbacks. It is a new implant consisting of a mono-block vulcanized silicone foam body, with no filling, which is like a sponge, similar to soap foam with multiple micro-cells, with a gelatin consistency on its surface for providing a soft and natural texture and with a greater density in its nucleus. In order to provide consistency to the compression and recovery of form due to body weight, a thin vulcanized silicone covering is added so that healing cells do not penetrate the structure of the prosthesis fixing it to the tissues and being capable of moving inside the women's breast.

Furthermore, this prosthesis provides advantages such as the impossibility of punctures or small ruptures when handled, since, although these do occur, since they lack fluid, the extravasation thereof for any reason is impossible, given that all its content is "solid" in a micro-cell arrangement, therefore preventing that, with time, this prosthesis loses volume, with its corresponding maintenance, costs, second operations, discomforts, etc.

Since it incorporates no fluid content, all those drawbacks derived from any product more or less irritating for the organism upon bursting disappear, eliminating callosities and hardness in the scar and wounds due to irritation with products such as liquid or gelatin silicone, PVP- polyvinylpyrrolidone, saline, hydrogel, triglycerides, etc, thus avoiding to the patient the need of new surgery with the subsequent advantages of health and economy.

The main cause of rupture and change of implant in the existing models is the formation of folds with the subsequent rupture due to friction in the peaks of the folds. With the present implant model, .even though the known folds are formed, only one of the micro-cells of this fold would break, without affecting the rest.

Since it is not a solid implant either and barely contributes to increasing the weight of the woman's breast, sagging of the feminine bust with years is much less than with current implants, having, at least, a much greater weight/volume ratio than the new proposed implant, which have a weight/volume ratio that is equal to or greater than the density of water per unit volume.

This implant is mono-block and mono-substance, in which there is only low density vulcanized silicone, which provides a high radiotransparency, significantly improving the high opacity of the current silicone gel implants; thus aiding in observing this area of the patient by means of x-rays, since the density of the material which the X-rays must penetrate is lower.

Losses of manufacturers due to manufacturing defects (pores, sealing leakages, etc) are completely eliminated, as well as changes of implants due to early rupture, defrayed by all prestigious manufacturers.

In turn, as vulcanized silicone, which is admitted for medical use by all current international standards (F.D.A., EC, etc.), is the only substance used, implying no risk, it implies approval by health institutions.

### DESCRIPTION OF THE DRAWINGS

To complement the description being made and for the purpose of helping to better understand the features of the invention, a set of drawings accompany the present specification as an integral part thereof which, with an illustrative and non-limiting character, show the following:

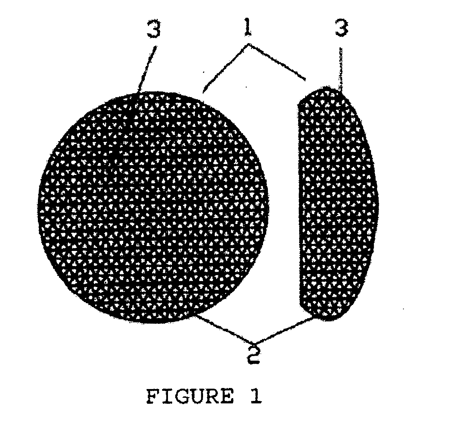

Figure 1.- Shows a profile elevational view of the prosthesis object of the present invention.

### PREFERRED EMBODIMENT OF THE INVENTION

As can be seen in the attached drawings, first a body (1) is distinguished, wrapped or not in a silicone covering (2) which is composed of a micro-cell structure (3), forming a solid and at the same time hollow structure; it can also be observed that the view of the profile of the prosthesis without a covering coincides with the cross sectional.view of said profile, since the cross section shows the same figure as multiple micro-cells adhered to one another, that is, an infinity of solidly joined micro-cells of about 2 to 4 mm (3), covered or not with a silicone covering (2).

Therefore, any sharp object penetrating inside the body of the prosthesis will not burst any membrane containing fluids inside, but rather, at most, it would scratch or perforate some walls of some micro-cells (3), then there is no product that can extravasate from its housing, thus preventing the decrease of volume as well as the encapsulation effect.

At the same time, to prevent the prosthesis from being crushed by body weight or pressure, in this prosthesis the density of the material used adopts a gradient of lesser to greater density from the outside towards the inside , such that the outer part has a more or less soft and smooth texture, and the inner part has a higher weight and density, therefore this inner part will be much more difficult to crush, maintaining a minimum volume in the prosthesis regardless of it being crushed.

From the aforementioned, a compact but at the same time light silicone material breast prosthesis is deduced, whenever the inside of the micro-cells (3) will preferably be hollow, in other words, incorporating air, being susceptible to introducing another compatible gas or substance.

Having sufficiently described the nature of the present invention, as well as an embodiment, all that is left to add is that it is possible to introduce changes of shape, materials and arrangement in its assembly and parts composing it as long as said alterations do not significantly change the features of the invention which are claimed below.

## Claims

1. A breast prosthesis of those which are introduced in the breast of a woman for aesthetic purposes, it consists of body (1), wrapped or not in a silicone covering (2) which is composed of a hollow micro-cells (3) structure, forming a solid and at the same time hollow structure, the material of which is of silicone, **characterized by,** the density of the prosthesis varying from lesser to greater from the outside towards the nucleus, which also implies a higher trabecular density and weight.

## Patentansprüche

1. Brustprothese, der Art, die für ästhetische Zwecke in die Brust einer Frau eingeführt wird, **dadurch gekennzeichnet, dass** sie aus einem Körper (1) besteht, der mit einer aus einer Struktur hohler Mikrokugeln (3) bestehenden Silikonhülle, die eine feste und gleichzeitig hohle Struktur bildet, deren Material aus Silikon besteht, umhüllt sein kann oder nicht, wobei die Dichte der Prothese von außen zum Kern ansteigt, was außerdem eine höhere trabekuläre Dichte und ein höheres Gewicht bedeutet.

## Revendications

1. Prothèse mammaire, du type de celles qui s'introduisent dans le sein de la femme au niveau esthétique, **caractérisée en ce qu'**elle est composée d'un corps (1), enveloppé ou non par une couverture (2) de silicone, laquelle est composée d'une structure de microsphères (3) creuses, qui conforme une structure solide et creuse à la fois, dont la matière est en silicone, la densité de la prothèse allant de plus faible à plus forte, depuis l'extérieur jusqu'au noyau, lequel suppose aussi une plus forte densité trabéculaire et de poids.
